# EUROPEAN PATENT APPLICATION

(11) **EP 2 172 561 A1**
(43) Date of publication of application: **07.04.2010**
(21) Application number: 08777360.2
(22) Date of filing: 12.06.2008
(51) Int. Cl.: C12Q 1/68, C12N 15/09, G01N 21/78, G01N 33/569

(54) **IMPROVED METHOD OF DETECTING NOROVIRUS RNA**

(30) Priority: 22.06.2007 JP 2007164921
(71) Applicant: Tosoh Corporation, Yamaguchi 746-8501 (JP)
(72) Inventor: MASUDA, Noriyoshi, Tokyo 153-0063 (JP); UNE, Kurando, Yokohama-shi Kanagawa 240-0022 (JP); SAITO, Juichi, Yamato-shi Kanagawa 242-0028 (JP); HAYASHI, Toshinori, Sagamihara-shi Kanagawa 228-0804 (JP)
(74) Representative: Müller-Boré & Partner Patentanwälte
(86) International application number: PCT/JP2008/061184
(87) International publication number: WO 2009/001737

(57) **Abstract**

The amount of an RNA transcription product amplified in an RNA amplification process is measured using a nucleic acid probe labeled with an intercalating fluorescent dye. The RNA amplification process comprises the steps of using at least two sets of primer pairs comprising a first primer and a second primer (in which one of these primers carries a promoter sequence added to the 5' end thereof), both of which have high hybridization efficiency to a nucleic acid sequence that is homologous to or complementary to each norovirus genotype RNA; forming a double-stranded DNA containing the promoter sequence with a reverse transcriptase; forming an RNA transcription product with an RNA polymerase by using the double-stranded DNA as a template; and forming the double-stranded DNA by successively using the RNA transcription product as a template in the DNA synthesis with the reverse transcriptase.

## Description

### Field of the Invention

The present invention relates to a simple and quick detection method for the norovirus, and it is useful for clinical, public health, food, and food poisoning testing.

### Prior Art

The norovirus is a member of the human calicivirus family, and has a genome consisting of a single-strand RNA of about 7000 bases. The norovirus is also referred to as a Small Round Structured Virus (SRSV).

Roughly 20% of cases of food poisoning reported in Japan are estimated to be caused by viruses. The norovirus is detected in about 80% of these cases of viral food poisoning. The main infection source is food, and raw oysters are frequently the problem. In addition, the norovirus has also been detected in (sporadic) acute gastroenteritis among infants, and the possibility of person-to-person propagation has been suggested. Since testing for the norovirus is an important issue in terms of public health and food quality control, there is a need for the development of a highly sensitive and rapid testing method capable of detecting all or most subtypes, using a gene amplification process. Previously, detection of the norovirus has been carried out based on observation by electron microscopy. This method can detect all subtypes, but requires 10⁶ cells/mL or more to detect the virus because of its low sensitivity, thus, specimens are limited to patient stool samples. Also, the virus can be observed by this method, but cannot be identified.

Further, an ELISA reagent using virus-like hollow particles of human calicivirus for detecting a specific antibody has been developed (see WO2000/079280). However, the detection sensitivity thereof is similar to that of using an electron microscopy method, and is not very high.

One of the means for highly sensitive measurement of the norovirus is a method of amplifying norovirus RNA by RT-PCR (see Japanese Patent No. 3,752,102), but this method generally requires a two-stage process consisting of a reverse transcription (RT) stage and a PCR stage, resulting in a complicated procedure and low reproducibility, as well as an increased risk of secondary contamination. The combined RT and PCR stages take two hours or longer in most cases, and the method is therefore unsuitable for processing of large-scale testing or reducing the cost of testing.

Assays of target RNA have commonly used the Real-Time RT-PCR method, whereby the PCR stage is carried out in the presence of an intercalating fluorescent dye and the increase in fluorescence is measured (see Kageyama T. et al., Journal of Clinical Microbiology, 41, 1548-1557 (2003)), but this method has a problem associated with detection of non-specific amplification products, such as primer dimers. In addition, PCR requires an abrupt increase and decrease in reaction temperatures, which creates obstacles with respect to power savings and cost reduction for automated reactors.

However, the NASBA method (see Japanese Patent Nos. 2,650,159 and 3,152,927) and TMA method (see Japanese Patent No. 3,241,717), etc., have been reported as methods for amplifying only RNA in an isothermal manner. These RNA amplification methods employ a chain reaction
wherein a primer including the promoter sequence for the target RNA, reverse transcriptase, and if necessary, Ribonuclease H (RNase H) are used for the synthesis of double-stranded DNA containing the promoter sequence, an RNA polymerase is used for the synthesis of RNA containing the specified nucleotide sequence of the target RNA, and the RNA is in turn used as a template for synthesis of double-stranded DNA containing the promoter sequence. After the RNA amplification, the amplified RNA is detected by electrophoresis or a hybridization method using a nucleic acid probe bound to a detectable label.

As stated above, these RNA amplification methods are suitable for simple RNA measurement since they amplify only RNA in an isothermal, single-stage manner, but detection by hybridization methods and the like require complex procedures and have a problem that highly reproducible quantitation is impossible.

A convenient method for the amplification and assay of mRNA is the method developed by Ishiguro et al. (see Japanese Unexamined Patent Publication No. 2000-14400 and Ishiguro, T. et al., Analytical Biochemistry, 314, 77-86 (2003)). In this method, RNA amplification is carried out in the presence of a nucleic acid probe, which is labeled with an intercalating fluorescent dye and is designed so that, when a complementary double strand with the target nucleic acid is formed, the intercalating fluorescent dye portion undergoes a change in fluorescent property by intercalating into the complementary double strand, and the change in fluorescent property is measured. It is possible to simultaneously accomplish RNA amplification and assay in a convenient, isothermal, and single-stage manner in a sealed vessel.

All of these methods for amplifying nucleic acids use a primer set comprising a sense primer (the first primer)and an anti-sense primer (the second primer) to amplify the target RNA, and it is well-known that the combination of primer sequences significantly affects the amplification efficiency and the specificity. However, since the norovirus has many variable genotypes, it has been difficult to construct a primer set which amplifies all norovirus genotypes with uniformity and high efficiency.

### Disclosure of the Invention

The norovirus is broadly divided into two types consisting of genogroup I (GI) and genogroup II (GII). Regarding both genotypes, GI is divided into 14 genotypes and GII is divided into seventeen genotypes at present (Refer to Infectious Diseases Weekly Report, 6 (11), 14-19 (2004)). The gene sequence homology among genotypes belonging to GI and among genotypes belonging to GII is about 70%. In addition, the homology between base sequences of GI and GII is about 40 to 50%.

In order to improve the detectability of a norovirus detection reagent used in a gene amplification process, regarding the regions to be used for primer bindings, there must be at least two regions of a length of at least 20 bases, each region having a base sequence which is common among all subtypes. However, having researched the homology of the following six sequences indicated as norovirus sequences in GenBank (Chiba (No. AB042808), Norwalk (No. M87661), Southampton (No. L07418), Camberwell (No. AF145896), Hawaii (No. U07611)), and HuCV (No. AY032605)), these are no regions of 20 or more bases in which the base sequence is the same among all genotypes.

Under these circumstances, the inventors of the present invention have provided norovirus detection methods which can detect a broad range of genotypes of GI and GII with high sensitivity (see Japanese Unexamined Patent Publication No. 2005-245434). However, it is still impossible to uniformly detect all genotypes of the norvirus with highly sensitivity by the methods, and thus, a highly sensitive detection method for a broader range of genotypes of norvirus RNA has been desired.

The inventors of the present invention conducted intensive research to solve the above problem. As a result, it is possible to detect a broader range of genotypes of norovirus RNA simply and rapidly with high-sensitivity.

According to the first invention, the present invention provides a method for detecting norovirus RNA in a sample, comprising:
(1) using a specified nucleic acid sequence derived from norovirus RNA as a template, producing a double-stranded DNA comprising a promoter sequence and the specified base sequence downstream from the promoter sequence, with a first primer and a second primer either one of which is added with the promoter sequence at the 5' end, an RNA-dependent DNA polymerase, a ribonuclease H (RNase H) and a DNA-dependent DNA polymerase;
(2) producing an RNA transcript comprised of the specified base sequence with RNA polymerase using the double-stranded DNA as a template;
(3) amplifying the RNA transcript in a chain reaction using the RNA transcript as a template for the subsequent double-stranded DNA synthesis; and
(4) measuring an amount of the RNA transcript,
wherein the first primer consists of a mixture of at least two kinds of oligonucleotides selected from the group of oligonucleotides each of which is sufficiently homologous to any one of the sequences listed as SEQ ID Nos. 1 to 4, and the second primer consists of at least two kinds of oligonucleotides selected from the group of oligonucleotides each of which is sufficiently complementary to any one of the sequences listed as SEQ ID Nos. 5 to 9.

The second invention relates to the method for detecting norovirus RNA of the first invention, wherein the first primer consists of a mixture of at least two kinds of oligonucleotides selected from the oligonucleotides listed as SEQ ID No. 10 which is a partial sequence of SEQ ID No. 1, SEQ ID No. 11 which is a partial sequence of SEQ ID No. 2, and SEQ ID No. 12 which is a partial sequence of SEQ ID No. 3, and the second primer consists of at least two kinds of oligonucleotides selected from the oligonucleotides listed as SEQ ID No. 13 which is a complementary sequence of SEQ ID No. 5; SEQ ID No. 14 which is a complementary sequence of SEQ ID No. 7, SEQ ID No. 15 which is a complementary sequence of SEQ ID No. 8, and SEQ ID No. 16 which is a complementary sequence of SEQ ID No. 9.

The third invention relates to the method for detecting norovirus RNA of the first invention, wherein the first primer consists of a mixture of at least two kinds of oligonucleotides selected from the group of oligonucleotides each of which includes 14 nucleotide continuous sequence which is homologous to any one of sequences listed as SEQ ID Nos. 1 to 4.

The fourth invention relates to the method for detecting norovirus RNA of the first invention, comprising the following steps:
by use a specified nucleic acid sequence derived from norovirus RNA as a template, cleaving norovirus RNA at the 5' end portion of the specific nucleic acid sequence with a cleavage oligonucleotide and RNase H,
wherein the cleavage oligonucleotide is complementary to the region adjacent to in the 5' direction and overlapping with the 5' end portion of the specified nucleic acid sequence which is homologous to the first primer, followed by producing the double-stranded DNA with the first primer added with a promoter sequence at the 5' end, the second primer, the RNA-dependent DNA polymerase, the RNase H and the DNA-dependent DNA polymerase; wherein the double-stranded DNA includes the promoter sequence and the specified base sequence downstream from the promoter sequence, wherein the cleavage oligonucleotide consists of a mixture of at least two kinds of oligonucleotides selected from the group of oligonucleotides each of which is sufficiently complementary to any one of SEQ ID Nos. 17 to 21.

The fifth invention relates to the method for detecting norovirus RNA of any one of the first to fourth inventions, wherein step (4) of measuring an amount of RNA transcript is carried out by measuring a signal change in the presence of a nucleic acid probe designed to change a signal property when the nucleic acid probe forms a complementary double-strand with the target RNA, and the nucleic acid probe consists of a sequence which is sufficiently complementary to SEQ ID No. 22.

### Brief Description of the Drawings

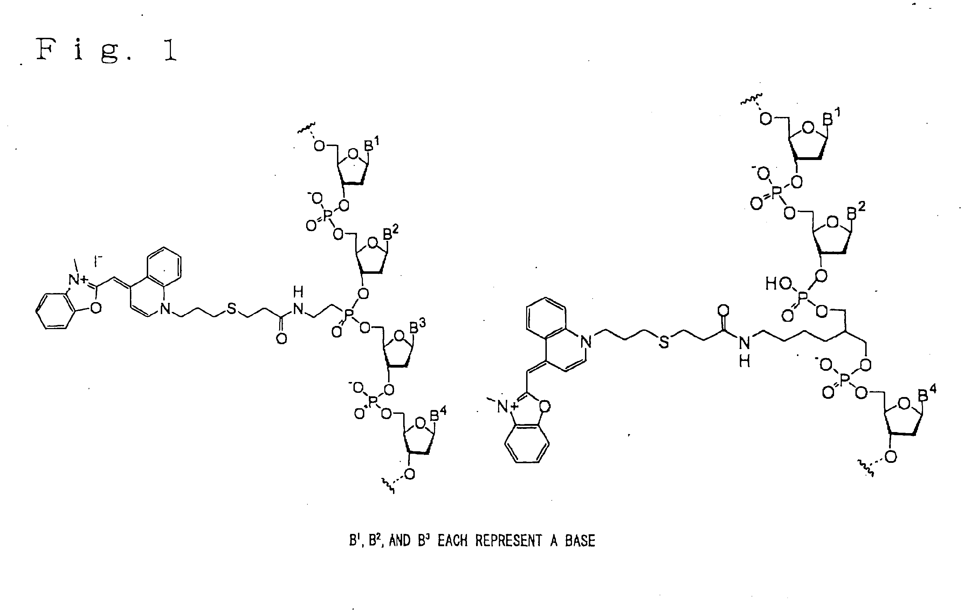

Fig. 1 shows the structure of the intercalating fluorescent dye-labeled nucleic acid probe prepared in Example 2. B1, B2, B3, and B4 each represent a base. A probe having intercalating fluorescent dye (oxazole yellow) is bonded via a linker to a phosphate diester portion according to the method of Ishiguro, T. et al. (Nucleic Acids Research, 24, 4992-4997 (1996)). In order to prevent 3'-terminal hydroxyl group extension reaction, the 3'-terminal hydroxyl group is modified with glycolic acid.

### Best Mode for Carrying Out the Invention

The following provides a detailed explanation of the present invention.

The specified base sequence according to the present invention means an RNA or DNA base sequence homologous to a norovirus RNA base sequence, from the 5' end region which is homologous to the first primer to the 3' end region which is complementary to the second primer. According to the present invention, the RNA transcript from the specified base sequence is amplified. The 5' end portion of the region homologous to the first primer according to the present invention consists of a partial sequence including the 5' end of the homologous region in the specified base sequence, and the partial sequence is an overlapping portion which includes a region complementary to the cleavage oligonucleotide and a region homologous to the first primer. The promoter according to the present invention is a sequence to which an RNA polymerase binds to initiate transcription, and the promoter sequences are known to be specific for different RNA polymerases. The RNA polymerase is not particularly limited, however, T7 promoter, SP6 promoter, T3 promoter, etc., are preferable from the viewpoint of the general usage in molecular biological experiments.

A sufficiently complimentary sequence according to the present invention means a sequence capable of hybridizing with the specified base sequence with specificity and high efficiency under the optimized conditions for a nucleic acid amplification reaction (salt concentration, oligonucleotide concentration, reaction temperature, etc.). A sufficiently homologous sequence according to the present invention means one capable of hybridizing with a complete complementary sequence of the specified base sequence with specificity and high efficiency under the optimized conditions for a nucleic acid amplification reaction (salt concentration, oligonucleotide concentration, reaction temperature, etc.). Accordingly, if the sufficiently complimentary or sufficiently homologous sequence according to the present invention is in the range where the hybridization specificity and efficiency are not adversely affected, the length, etc., of the sequence can be arbitrarily designed. There are no particular restrictions, but a sufficiently complimentary sequence according to the present invention may be preferably an oligonucleotide including at least 14 continuous bases of a sequence completely complementary to the specified base sequence. Also, there are no particular restrictions, but a sufficiently homologous sequence according to the present invention may be preferably an oligonucleotide including at least 14 continuous bases of a sequence completely homologous to the specified base sequence. Further, the optimized reaction conditions for a nucleic acid amplification reaction are not particularly restricted, but may be as indicated in the examples described below.

As described above, at present, norovirus GI RNA has 14 genotypes and norovirus GII RNA has 17 genotypes, and a highly conserved region in the base sequence does not have sufficient length. Thus, it has been difficult to detect all genotypes with high sensitivity using one set of the combination of a first primer and a second primer. According to the present invention, the inventors constructed a detection method using at least two primer combinations of the first and second primers which are sufficiently homologous or complementary to a plurality of genotypes, thereby, a highly sensitive detection method for broader range of genotypes is possible.

In the invention, the first primer for detecting norovirus RNA is an oligonucleotide mixture comprising at least two kinds of oligonucleotides selected from the group of oligonucleotides each of which is sufficiently homologous to any one of the sequences listed as SEQ ID Nos. 1 to 4, and the second primer is an oligonucleotide mixture comprising at least two kinds of oligonucleotides selected from the group of oligonucleotides each of which is sufficient complementary to any one of the sequences listed as SEQ ID Nos. 5 to 9.

More preferably, the first primer is an oligonucleotide mixture comprising at least two kinds of oligonucleotides selected from the group of oligonucleotides listed as SEQ ID Nos. 10 to 12, and the second primer is an oligonucleotide mixture comprising at least two kinds of oligonucleotides selected from the group of oligonucleotides listed as SEQ ID Nos. 13 to 16.

As one embodiment of the present invention, the first primer is comprised of the sequences listed as SEQ ID Nos. 31, 32, and 33, and the second primer is comprised of the sequences listed as SEQ ID Nos. 34 and 35.

In the invention, norovirus RNA is cleaved at the 5' end portion of the specific nucleic acid sequence before serving as the template for cDNA synthesis. Due to the cleavage at the 5' end portion of the specified nucleic acid sequence, after cDNA synthesis, a DNA chain complementary to the promoter sequence of the first primer which is hybridized with cDNA can be efficiently synthesized by extending to the 3' end of the cDNA, resulting in the formation of a functional double-stranded DNA promoter structure. As a cleavage method, a method consisting of cleaving the norovirus RNA, with enzymes having ribonuclease H (RNase H) activity or the like, at the RNA portion of an RNA-DNA hybrid formed by adding an oligonucleotide (hereinafter, referred to as cleavage oligonucleotide) having a sequence complementary to the region adjacent in the 5' direction to and overlapping with the 5' end portion of the specific nucleic acid sequence in norovirus RNA is known (the partial sequence including the 5' end portion of the specific nucleic acid sequence). The hydroxyl group at the 3' end of the cleavage oligonucleotide, which is appropriately modified, for example, aminated, is preferably used in order to prevent an elongation reaction from the 3' end.

In order to cleave the 5' end portion with high efficiency, it is necessary to increase the hybridization efficiency of the cleavage oligonucleotide. However, as there are various genotypes in norovirus RNA, it is difficult to carry out highly efficient hybridization of a single cleavage oligonucleotide with the various genotypes.

In the invention, the cleavage oligonucleotide is a mixture comprised of at least two kinds of oligonucleotides selected from the group of cleavage oligonucleotides each having a sequence sufficiently complementary to any of the sequences listed as SEQ ID Nos. 17 to 21, more preferably the cleavage oligonucleotide is a mixture comprised of at least two kinds of oligonucleotides selected from the group of cleaving nucleotides comprised of at least 24 continuous oligonucleotides, each of which are complementary to at least of the SEQ ID No. 23 which is a partial sequence of the complementary sequence of SEQ ID No. 17, SEQ ID No. 24 which is a partial sequence of the complementary sequence of SEQ ID No. 18, SEQ ID No. 25 which is a partial sequence of the complementary sequence of SEQ ID No. 19 and SEQ ID No. 26 which is a partial sequence of the complementary sequence of SEQ ID No. 20. Using the mixture, the hybridization efficiency of the cleavage oligonucleotide is increased, and thereby a highly sensitive detection of a broader range of norovirus RNA genotypes is possible.

The target RNA according to the present invention refers to a region in the specified base sequence of the RNA transcript that is not homologous or complementary to the primers, while having a sequence that allows complementary binding with the intercalating fluorescent dye-labeled nucleic acid probe. Thus, the intercalating fluorescent dye-labeled nucleic acid probe is a sequence complementary to a portion of the specified base sequence according to the present invention. Therefore, according to one embodiment of the invention, a nucleic acid probe which is sufficiently complementary to SEQ ID No. 26 can be used as an intercalating fluorescent dye-labeled nucleic acid probe.

As one embodiment of the present invention, the cleavage oligonucleotide consists of sequences each of which is sufficiently complementary to any one of SEQ ID Nos. 17 to 21, and is sufficiently complimentary to a norovirus RNA (of the corresponding genotype). The first primer has a promoter sequence at the 5' end and is comprised of at least two oligonucleotide sequences, each of which are sufficiently complementary to any one of the sequences listed as SEQ ID Nos. 1 to 4, and also sufficiently complementary to a complete complementary sequence of the norovirus RNA (of the corresponding genotype). The second primer is comprised of at least two oligonucleotide sequences each of which is sufficiently complementary to any one of the sequences listed as SEQ ID Nos. 5 to 9, and is sufficiently complementary to a complete complementary sequence of the norovirus RNA (of the corresponding genotype). The intercalating fluorescent dye-labeled nucleic acid probe has a sequence which is sufficiently complimentary to SEQ ID No. 22 and is also sufficiently complementary to the target nucleic acid.

It is more preferable to use at least two oligonucleotides selected from the group of oligonucleotide sequences listed as SEQ ID Nos. 28 to 30 as the cleavage oligonucleotide; at least two oligonucleotides selected from the group of oligonucleotide sequences listed as SEQ ID Nos. 10 to 12 as the first primer; at least two oligonucleotides selected from the group of oligonucleotide sequences listed as SEQ ID Nos. 13 to 16 as the second primer; and an oligonucleotide sequence listed as SEQ ID No. 27 as the intercalating fluorescent dye-labeled nucleic acid probe.

The method for detecting norovirus RNA according to the present invention requires certain enzymes (an enzyme having RNA-dependent DNA polymerase activity for single-stranded RNA template, i.e., reverse transcriptase, an enzyme having RNase H activity, an enzyme having DNA-dependent DNA polymerase activity for single-stranded RNA template, and an enzyme having RNA polymerase activity). Any of these enzymes may be enzymes having different activities, or a plurality of enzymes having each activity may be used. For example, an enzyme having RNA-dependent DNA polymerase activity may be added to a reverse transcriptase having RNA-dependent DNA polymerase activity for single-stranded RNA template, RNase H activity and DNA-dependent DNA polymerase activity for single-stranded DNA template, or an additional enzyme with RNase H activity may be further added as a supplement. As the reverse transcriptase, enzymes commonly used in molecular biological experiments, such as AMV reverse transcriptase, MMLV reverse transcriptase, HIV reverse transcriptase, and their derivatives are preferable, particularly, and AMV reverse transcriptase and the derivatives are most preferable. Also, as the enzyme having RNA polymerase activity, enzymes derived from bacteriophages widely used in the molecular biological experiments such as the T7 RNA polymerase, T3 RNA polymerase, SP6 RNA polymerase, and their derivatives may be used.

As one embodiment of the present invention, the cleavage oligonucleotide is added to norovirus RNA in the sample, and cuts RNA at the 5' end portion of the specified base sequence by its RNase H activity. When the reverse transcription reaction is carried out using the cleaved RNA as template in the presence of the first primer and the second primer, the second primer binds to the specified base sequence of norovirus RNA and carries out cDNA synthesis with the enzyme having RNA-dependent DNA polymerase activity. The RNA portion of the obtained RNA-DNA hybrid is degraded by the enzyme having RNase H activity, and dissociates so that the first primer may bind to the cDNA. Next, double-stranded DNA derived from the specified base sequence and containing the promoter sequence at the 5' end is produced by the enzyme having DNA-dependent DNA polymerase activity. The double-stranded DNA contains the specified base sequence downstream from the promoter sequence, and an RNA transcript serves as template for the double-stranded DNA synthesis by the first and second promoters, so that a series of reactions occur in a chain reaction and result in amplification of the RNA transcript.

In order to promote the chain reaction, it is obviously necessary to add, at least, a buffering agent, magnesium salt, potassium salt, nucleoside triphosphates, and ribonucleoside triphosphates as known elements essential for each of the enzymes. In addition, additives such as dimethylsulfoxide (DMSO), dithiothreitol (DTT), bovine serum albumin (BSA), sugars, and the like can be added to control the reaction efficiency.

For example, when using AMV reverse transcriptase and T7 RNA polymerase, the reaction temperature is preferably set in a range of 35 to 65 °C, and most preferably it is set in a range of 40 to 44 °C. The RNA amplification step proceeds isothermally, and the reaction temperature can be set to any desired temperature at which the reverse transcriptase and RNA polymerase exhibit their activities.

The amount of amplified RNA transcript can be measured by a known nucleic acid assay method. As such assay methods, methods employing electrophoresis or liquid chromatography, or hybridization methods employing nucleic acid probes labeled with detectable labels may be used. However, these procedures involves multiple steps, and because the amplification product is removed out of the system for analysis, there is a high risk of secondary contamination caused by spreading the amplification product into the environment. To overcome these problems, it is preferable to use a nucleic acid probe designed so that its fluorescent property changes upon complementary binding to the target nucleic acid. As a more preferred method, there may be mentioned a method wherein the nucleic acid amplification step is carried out in the presence of a nucleic acid probe which is labeled with an intercalating fluorescent dye and is designed so that when it forms a complementary double strand with the target nucleic acid, the intercalating fluorescent dye portion undergoes a change in fluorescent property by intercalating into the complementary double strand, and the change in fluorescent property is measured (see Japanese Unexamined Patent Publication No. 2000-14400 and Ishiguro, T. et al. (2003), shown above).

There are no particular restrictions on the intercalating fluorescent dye, and common dyes such as oxazole yellow, tiazole orange, ethidium bromide and their derivatives may be used. The change in fluorescent property may be a change in fluorescent intensity. For example, it is known that in the case of oxazole yellow, intercalation into double-stranded DNA causes a notable increase in fluorescence at 510 nm (excitation wavelength of 490 nm). The intercalating fluorescent dye-labeled nucleic acid probe is an oligonucleotide which is sufficiently complementary to the target RNA in the RNA transcript, and it has a structure wherein the intercalating fluorescent dye is bonded via an appropriate linker to the end portion, a phosphate diester portion, or a base portion of the oligonucleotide, and the 3'-terminal hydroxyl group of the nucleotide is suitably modified to prevent extension from the 3'-terminal hydroxyl group (see Japanese Unexamined Patent Publication No. 8-211050 and Ishiguro, T. et al., (1996), shown above).

Labeling of the oligonucleotide with the intercalating fluorescent dye may be accomplished by introducing a functional group into the oligonucleotide by a known method and bonding the intercalating fluorescent dye thereto (see Japanese Unexamined Patent Publication No. 2001-13147 and Ishiguro, T. et al. (1996), shown above). The method of introducing the functional group may employ the commonly used Label-ON Reagents (Clontech Laboratories, Inc.).

As one embodiment of the invention, there is provided a method of adding to the sample an amplification reagent containing at least a first primer containing the T7 promoter sequence at the 5' end (sequence listed as SEQ ID Nos. 10, 11, or 12, added with the T7 promoter sequence (sequence listed as SEQ ID No. 58) at the 5' end thereof), a second primer (sequences listed as SEQ ID Nos. 15 and 16), an intercalating fluorescent dye-labeled nucleic acid probe (the sequence listed as SEQ ID No. 27), cleavage oligonucleotide (sequences listed as SEQ ID Nos. 28, 29, and 30), AMV reverse transcriptase, T7 RNA polymerase, buffering agent, magnesium salt, potassium salt, nucleoside triphosphates, ribonucleoside triphosphates, and dimethylsulfoxide (DMSO), and performing the reaction at a constant reaction temperature of 35 to 65°C (preferably 40 to 44°C) while periodically measuring the fluorescent intensity of the reaction solution.

As a different embodiment, there is provided a method of adding to the sample an amplification reagent containing at least a first primer containing the T7 promoter sequence (sequence listed as SEQ ID No. 58) at the 5' end (sequences listed as SEQ ID Nos. 31, 32, and 33), a second primer (sequences listed as SEQ ID Nos. 34 and 35), an intercalating fluorescent dye-labeled nucleic acid probe (the sequence listed as SEQ ID No. 27), a cleavage oligonucleotide (sequences listed as SEQ ID Nos. 36, 37, and 38), AMV reverse transcriptase, T7 RNA polymerase, buffering agent, magnesium salt, potassium salt, nucleoside triphosphates, ribonucleoside triphosphates, and dimethylsulfoxide (DMSO), and performing the reaction at a constant reaction temperature of 35 to 65°C (preferably 40 to 44°C) while periodically measuring the fluorescent intensity of the reaction solution.

In all of the embodiments mentioned above, since the fluorescent intensity is periodically measured, the measurement may be concluded at any desired point at which a significant increase in fluorescence is detected, and measurement results from the nucleic acid amplification and assay can be obtained usually within an hour.

It is especially notable that all of the test materials in the assay reagent can be included in the same vessel. That is, the simple procedure of dispensing prescribed amounts of test materials into a single vessel will allow the automatic amplification and detection of norovirus RNA to be conducted thereafter. The vessel may be constructed of, for example, a partially transparent material to allow external measurement of the signal emitted by the fluorescent dye, and a vessel that can be sealed after dispensing the test materials is particularly preferred to prevent contamination.

The RNA amplification and assay method according to the embodiments described above can be carried out in a single-stage and isothermal manner, and it is therefore more convenient than RT-PCR and is suitable for automation. According to the present invention, it is possible to assay a broad range of genotypes of norovirus RNA with high specificity, high sensitivity in a rapid, convenient, isothermal, and single-stage manner.

### EXAMPLE

Although the following provides a more detailed explanation of the invention of the present application through examples, the present invention is not limited by these examples.

### EXAMPLE 1

Norovirus RNA (hereinafter, referred to as standard RNA) used in Examples of the present application was prepared by the methods (1) and (2).
(1) Among the norovirus cDNA base sequences registered in the GenBank, double-stranded DNA of the base sequence regions of the genotypes as listed in Table 1 were prepared (SP6 promoter was added to the 5' end of the DNA).
(2) In vitro transcription using SP6 RNA polymerase was carried out using DNA prepared in Step (1) as a template, and the double-stranded DNA was completely digested by DNaseI treatment, and then, the RNA was purified. The RNA was quantitated by measuring the absorbance at 260 nm.

**Table 1**

| Genotype | Name | GenBank No. | Base Sequence Region |
|---|---|---|---|
| GI/1 | Norwalk | M87661 | 5113-5637 |
| GI/2 | Southampton | L07418 | 5110-5634 |
| GI/3 | Desert Shield | U04469 | 555-1079 |
| GI/4 | Chiba | AB042808 | 5101-5625 |
| GI/7 | Saitama T59 | AB112114 | 16-540 |
| GI/8 | WUG1 | AB081723 | 5110-5634 |
| GI/9 | Saitama SzU | AB078334 | 1346-1870 |
| GI/14 | Saitama T25 | AB112100 | 16-540 |

The total length of the standard RNA is 533 bases (8 bases derived from SP6 promoter were added at the 5' end of the RNA). Although it is only a partial portion of the total length of the norovirus RNA (about 7000 bases), it is sufficiently applicable for detecting the norovirus RNA, i.e., the measuring object of the present invention. Also, there were 8 prepared standard RNA genotypes, which is a partial portion of the total 14 genotypes of norovirus GI RNA, but the homology is high among the respective genotypes in (A) to (C) (see Infectious Diseases Weekly Report (IDWR), 6 (11), 14-19 (2004)),
(A) GI/1, GI/6, and GI/8
(B) GI/4, GI/5, and GI/9
(C) GI/3, GI/10, GI/11, GI/12, GI/13, and GI/14(1)
and thus, if it is possible to detect all of the prepared standard RNA, it can be assumed that all the genotypes of norovirus GI RNA can be detected.

### EXAMPLE 2

Oligonucleotide probes labeled with an intercalating fluorescent dye were prepared. According to the method described in Ishiguro, T. et al. (1996), oxazole yellow-labeled nucleic acid probes were prepared to have oxazole yellow bonded via a linker to the phosphate diester portion between the 12th C and the 13th A from the 5' end of SEQ ID No. 27 and the 12th C and the 13th A from the 5' end of SEQ ID No. 57 (Fig. 1).

### EXAMPLE 3

Using the combinations of the first primer, the second primer, an intercalating fluorescent dye-labeled nucleic acid probe (hereinafter, referred to as INAF probe), and cleavage oligonucleotide as shown in Table 2, the standard RNA was measured by methods (1) to (4). Regarding the combinations shown in Table 2, Combination A comprising the first primer, the second primer, and the cleavage oligonucleotide has the same sequences as described in Example 2 of Japanese Unexamined Patent Publication No. 2005-245434. Also, SEQ ID 10, 31, 40, 43, and 45 are respectively partial sequences of SEQ ID No. 1; SEQ ID No. 11, 32, 41, and 46 are respectively partial sequences of SEQ ID No. 2; SEQ ID No. 12, 33, and 47 are respectively partial sequences of SEQ ID No. 3; SEQ ID No. 42 and 44 are respectively partial sequences of SEQ ID No. 4; SEQ ID No. 34 is a partial sequence of the complementary sequence of SEQ ID No. 5; SEQ ID No. 48 is a sequence of the complementary sequence of SEQ ID No. 6; SEQ ID No. 35 is a partial sequence of the complementary sequence of SEQ ID No. 7; SEQ ID No. 15 is a sequence of the complementary sequence of SEQ ID No. 8; SEQ ID No. 16 is a sequence of the complementary sequence of SEQ ID No. 9; SEQ ID No. 28, 36, 51, and 54 are respectively partial sequences of the complementary sequence of SEQ ID No. 17; SEQ ID No. 29, 52, and 55 are respectively partial sequences of the complementary sequence of SEQ ID No. 18; SEQ ID No. 37 is a partial sequence of the complementary sequence of SEQ ID No. 19; SEQ ID No. 30, 38, and 56 are respectively partial sequences of the complementary sequence of SEQ ID No. 20; SEQ ID No. 53 is a partial sequence of the complementary sequence of SEQ ID No. 21; SEQ ID Nos. 27 and 57 are respectively partial sequences of the complementary sequence of SEQ ID No. 22.

**TABLE 2**

| Oligonucleotide Combination | Oligonucleotide [SEQ ID No.] | | | | | | | | | |
|---|---|---|---|---|---|---|---|---|---|---|
| | Cleaving Oligonucleotide | | | First Primer | | | Second Primer | | | INAF Probe |
| A | 50 | - | - | 39 | - | - | 49 | - | - | 57 |
| B | 51 | - | - | 40 | - | - | 15 | - | - | 57 |
| C | 52 | - | - | 41 | - | - | 48 | - | - | 57 |
| D | 53 | - | - | 42 | - | - | 16 | - | - | 57 |
| E | 51 | 53 | - | 40 | 42 | - | 15 | 16 | - | 57 |
| F | 52 | 53 | - | 41 | 42 | - | 16 | 48 | - | 57 |
| G | 51 | 52 | 53 | 40 | 42 | - | 15 | 16 | - | 57 |
| H | 51 | 53 | - | 40 | 41 | 42 | 15 | 16 | - | 57 |
| I | 51 | 53 | - | 40 | 42 | - | 15 | 16 | 48 | 57 |
| J | 51 | 52 | 53 | 40 | 41 | 42 | 15 | 16 | 48 | 57 |
| K | 50 | 52 | 53 | 39 | 41 | 42 | 16 | 48 | 49 | 57 |
| L | 51 | 52 | 53 | 40 | 42 | - | 34 | 35 | - | 57 |
| M | 54 | 55 | - | 43 | 44 | - | 34 | 35 | - | 57 |
| N | 54 | 55 | - | 43 | 44 | - | 15 | 16 | - | 57 |
| O | 54 | 55 | 56 | 43 | 44 | - | 34 | 35 | - | 57 |
| P | 54 | 55 | 56 | 43 | 44 | - | 15 | 16 | - | 57 |
| Q | 28 | 29 | 30 | 10 | 11 | 12 | 34 | 35 | - | 27 |
| R | 28 | 29 | 30 | 10 | 11 | 12 | 15 | 16 | - | 27 |
| S | 28 | 29 | 30 | 45 | 46 | 47 | 34 | 35 | - | 27 |
| T | 36 | 37 | 38 | 31 | 32 | 33 | 34 | 35 | - | 27 |
| U | 36 | 37 | 38 | 31 | 32 | 33 | 15 | 16 | - | 27 |
| V | 36 | 37 | - | 31 | 32 | 33 | 34 | 35 | - | 27 |
| W | 36 | 38 | - | 31 | 32 | 33 | 34 | 35 | - | 27 |
| X | 36 | 37 | 38 | 31 | 32 | - | 34 | 35 | - | 27 |

(1) Each standard RNA was diluted to 10³ copies/5 µL using an RNA diluent (10 mM Tris-HCl (pH 8.0), 1 mM EDTA, 0.25 U/µL ribonuclease inhibitor, 5.0 mM DTT) for use as an RNA sample.
(2) 20 µL portion of a reaction solution having the composition shown below was dispensed into a 0.5 mL volume PCR tube (Gene Amp Thin-Walled Reaction Tubes, Applied Biosystems, Inc.), and 5 µL of the RNA sample was added.
   Reaction solution composition: Final concentrations and amounts after addition of the enzyme solution (30 µL)
   60 mM of Tris-HCl (pH 8.6)
   18 mM of magnesium chloride
   100 mM of potassium chloride
   1 mM of DTT
   0.25 mM of dATP, dCTP, dGTP, dTTP each
   3 mM of ATP, CTP, UTP, GTP each
   3.6 mM of ITP
   0.75 µM of the first primer: the first primer included the T7 promoter sequence (SEQ ID No. 58) added to the 5' end of the indicated base sequence
   1 µM of the second primer
   20 nM of an intercalating fluorescent dye-labeled nucleic acid probe (INAF probe): The nucleic acid probe was prepared in Example 2
   0.16 µM of cleavage oligonucleotide: The 3' end hydroxyl group of the oligonucleotide was modified with an amino group
   6 U of ribonuclease inhibitor (Takara Bio Inc.)
   13% DMSO
(3) This reaction solution was incubated at 43°C for 5 minutes, and then 5 µL of the enzyme solution having the following composition and preheated at 43°C for 2 minutes was added.
   Enzyme solution composition: Final concentrations and amount during reaction (30 µL)
   2% sorbitol
   6.4 U of AMV reverse transcriptase (Life Science Co., Ltd.)
   142 U of T7 RNA polymerase (Invitrogen Corp.)
   3.6 µg of bovine serum albumin
(4) The reaction was then carried out at 43°C while periodically measuring the fluorescent intensity of the reaction solution (excitation wavelength: 470 nm, fluorescent wavelength: 520 nm) for 60 minutes using a fluorescent spectrometer equipped with a heat regulating function, capable of direct measurement of the PCR tube.

The time when an enzyme was added is defined as 0. When the fluorescent intensity ratio (fluorescent intensity value at a prescribed time/background fluorescent intensity value) of the reaction solution exceeded 1.2, the test as positive and the time was defined as the detection time. The results of the detection time are shown in Table 3. In Table 3, N.D. indicates that the fluorescent intensity ratio 60 minutes after enzyme addition was less than 1.2 (negative).

**TABLE 3**

| Oligonucleotide Combination | Detection Time [minutes] (10^3 copies/test) | | | | | | | |
|---|---|---|---|---|---|---|---|---|
| | Standard RNA Genotype | | | | | | | |
| | GI/1 | GI/2 | GI/3 | GI/4 | GI/7 | GI/8 | GI/9 | GI/14 |
| A | 11.4 | 51.9 | N. D. | 8.6 | N. D. | N.D. | N.D. | N.D. |
| B | 10.9 | 14.1 | N.D. | 9.9 | N.D. | N.D. | N.D. | N.D. |
| C | 15.3 | 8.8 | N.D. | 12.4 | N.D. | 12.3 | N.D. | 29.2 |
| D | N.D. | N.D. | 16.4 | 13.2 | 15.5 | 21.6 | N.D. | 10.4 |
| E | 13.8 | 20.4 | 16.5 | 12.1 | 13.8 | 33.4 | N.D. | 13.0 |
| F | 21.6 | 9.6 | 18.3 | 13.7 | 11.2 | 13.9 | N.D. | 11.0 |
| G | 11.8 | 13.6 | 19.9 | 11.1 | 15.9 | 23.8 | 25.4 | 13.2 |
| H | 12.6 | 12.1 | 17.5 | 11.4 | 14.8 | 23.4 | 25.3 | 13.2 |
| I | 11.8 | 23.1 | 18.6 | 11.0 | 15.3 | N. D. | 31.7 | 14.2 |
| J | 13.0 | 10.9 | 16.7 | 10.7 | 17.3 | 32.7 | N.D. | 15.5 |
| K | 51.1 | 13.9 | 24.1 | 13.9 | 15.2 | 22.5 | N.D. | 16.6 |
| L | 11.6 | 13.7 | 16.0 | 12.0 | 16.3 | 47.4 | N.D. | 11.6 |
| M | 17.0 | 21.0 | 19.2 | 34.5 | 38.1 | 28.2 | 18.5 | 27.8 |
| N | 16.9 | 38.6 | 24.9 | 15.7 | 49.1 | N. D. | 17.3 | 13.7 |
| O | 16.5 | 15.6 | 14.6 | 15.2 | 56.2 | 18.4 | 37.1 | 12.6 |
| P | 16.3 | 18.6 | 14.6 | 14.5 | 24.5 | 18.3 | 28.4 | 13.6 |
| Q | 13.6 | 10.3 | 10.6 | 12.1 | 11.2 | 15.5 | 12.2 | 12. 1 |
| R | 15.2 | 10.2 | 11.0 | 11.4 | 11.7 | 12.7 | 13.9 | 12.0 |
| S | 18.5 | 19.0 | 24.3 | 23.0 | 24.9 | 24.6 | N.D. | 26.3 |
| T | 12.7 | 10.9 | 11.0 | 11.8 | 11.9 | 12.0 | 17.6 | 11.2 |
| U | 13.0 | 11.1 | 12.9 | 13.1 | 11.4 | 13.4 | 15.4 | 13.0 |
| V | 13.6 | 11.6 | 12.2 | 14.3 | 16.5 | 25.8 | 14.1 | 15.0 |
| W | 15.0 | 11.4 | 10.9 | 15.2 | 13.2 | 21.8 | 15.2 | 13.1 |
| X | 17.8 | 14.2 | 11.6 | 12.7 | 12.7 | N. D. | 14.4 | 12.0 |

The oligonucleotide combinations A, B, G, I, and L resulted in rapid detection of the standard RNA derived from GI/1 (Norwalk), which is a major genotype of norvirus GI RNA. On the other hand, the oligonucleotide combinations Q, R, T, and U were preferable for the rapid detection of the standard RNA derived from a broad range of genotypes of norovirus GI RNA, and specifically, the most preferable combination was Combination T.

Further, the combinations (Combinations E to L) comprised of at least two first primers, second primers, and cleavage oligonucleotides respectively in each resulted in the detection of the standard RNA derived from a broader range of norovirus genotypes than the combinations (Combinations A to D) comprised of one kind of the first primer, the second primer, and cleavage oligonucleotide. From this, it was found to be very useful to use at least two kinds of each of the primers or the like in order to detect a broader range of norovirus genotypes with high sensitivity.

Moreover, there was almost no difference between use of the first primers which are oligonucleotides having the length of 20 (Combinations Q and R) and use of the first primers which are oligonucleotides having the length of 14 (Combinations T and U) in the detection of the standard RNA derived from each genotype among Combinations Q, R, T, and U. Therefore, it was concluded that the sufficient length of the first primer is 14 nucleotides.

As explained above, the RNA amplification and detection method using the first primer, the second primer, the nucleic acid probe labeled with an intercalating fluorescent dye, and the cleavage oligonucleotide according to the present invention is effective for detecting a broad range of genotypes of norovirus GI RNA with high sensitivity.

## Claims

1. A method for detecting norovirus RNA in a sample, comprising the following steps:
(1) by use of a specified nucleic acid sequence derived from norovirus RNA as a template, producing a double-stranded DNA comprising a promoter sequence and said specified base sequence downstream from the promoter sequence, with a first primer and a second primer either one of which is added with the promoter sequence at the 5' end, an RNA-dependent DNA polymerase, a ribonuclease H (RNase H) and a DNA-dependent DNA polymerase;
(2) producing an RNA transcript comprised of the specified base sequence with RNA polymerase using said double-stranded DNA as a template;
(3) amplifying the RNA transcript in a chain reaction using the RNA transcript as a template for the subsequent double-stranded DNA synthesis; and
(4) measuring an amount of the RNA transcript,
wherein the first primer consists of a mixture of at least two kinds of oligonucleotides selected from the group of oligonucleotides each of which is sufficiently homologous to any one of the sequences listed as SEQ ID Nos. 1 to 4, and the second primer consists of at least two kinds of oligonucleotides selected from the group of oligonucleotides each of which is sufficiently complementary to any one of the sequences listed as SEQ ID Nos. 5 to 9.

2. The method for detecting norovirus RNA according to claim 1, wherein the first primer consists of a mixture of at least two kinds of oligonucleotides selected from the oligonucleotides listed as SEQ ID No. 10, which is a partial sequence of SEQ ID No. 1, SEQ ID No. 11, which is a partial sequence of SEQ ID No. 2, and SEQ ID No. 12, which is a partial sequence of SEQ ID No. 3, and
the second primer consists of at least two kinds of oligonucleotides selected from the oligonucleotides listed as SEQ ID No. 13, which is a complementary sequence of SEQ ID No. 5, SEQ ID No. 14, which is a complementary sequence of SEQ ID No. 7, SEQ ID No. 15, which is a complementary sequence of SEQ ID No. 8, and SEQ ID No. 16, which is a complementary sequence of SEQ ID No. 9.

3. The method for detecting norovirus RNA according to claim 1, wherein the first primer consists of a mixture of at least two kinds of oligonucleotides selected from the group of oligonucleotides each of which includes 14 nucleotide continuous sequence, which is homologous to any one of sequences listed as SEQ ID Nos. 1 to 4.

4. The method for detecting norovirus RNA according to any one of claims 1 to 3, comprising the following steps:
by use a specified nucleic acid sequence derived from norovirus RNA as a template, cleaving norovirus RNA at the 5' end portion of the specific nucleic acid sequence with a cleavage oligonucleotide and RNase H, , wherein the cleavage oligonucleotide is complementary to the region adjacent to in the 5' direction and overlapping with the 5' end portion of the specified nucleic acid sequence which is homologous to the first primer, followed by producing the double-stranded DNA with the first primer added with a promoter sequence at the 5' end, the second primer, the RNA-dependent DNA polymerase, the RNase H and the DNA-dependent DNA polymerase; wherein the double-stranded DNA includes the promoter sequence and the specified base sequence downstream from the promoter sequence,
wherein the cleavage oligonucleotide consists of a mixture of at least two kinds of oligonucleotides selected from the group of oligonucleotides each of which is sufficiently complementary to any one of SEQ ID Nos. 17 to 21.

5. The method for detecting norovirus RNA according to any of claims 1 to 4, wherein step (4) of measuring an amount of RNA transcript is carried out by measuring a signal change in the presence of a nucleic acid probe designed to change a signal property when the nucleic acid probe forms a complementary double-strand with the target RNA, and the nucleic acid probe consists of a sequence which is sufficiently complementary to SEQ ID No. 22.


SEQUENCE LISTING

<110> TOSOH CORPORATION

<120> Improved Method for Detecting Norvirus

<130> V623-PCT

<150> JP 2007-164921
<151> 2007-06-22

<160> 58

<210> 1
<211> 65
<212> DNA
<213> Artificial sequence

<220>
<223> First Primer

<400> 1
gatgcgcttc catgacctcg gattgtggac aggagatcgc gatcttctgc 65
ccgaattcgt aaatg

<210> 2
<211> 65
<212> DNA
<213> Artificial sequence

<220>
<223> First Primer

<400> 2
gatgcggttc catgaccttg gtttgtggac aggagatcgc aatctcctgc 65

ccgaatttgt aaatg

<210> 3
<211> 65
<212> DNA
<213> Artificial sequence

<220>
<223> First Primer

<400> 3
gatgcgcttc catgatctga gcatgtggac aggggatcgc gatctcctgc 65
ccgattatgt aaatg

<210> 4
<211> 65
<212> DNA
<213> Artificial sequence

<220>
<223> First Primer

<400> 4
gatgcgattc catgatttga gcttgtggac aggagaccgc gatctcttgc 65
ccgattatgt aaatg

<210> 5
<211> 18
<212> DNA
<213> Artificial sequence

<220>
<223> Second Primer

<400> 5
attgatccct ggataatt                                                    18


<210> 6
<211> 18
<212> DNA
<213> Artificial sequence

<220>
<223> Second Primer

<400> 6
attgatccct ggattgtt                                                    18


<210> 7
<211> 18
<212> DNA
<213> Artificial sequence

<220>
<223> Second Primer

<400> 7
attgacccct ggataatg                                                    18


<210> 8
<211> 18
<212> DNA
<213> Artificial sequence

<220>
<223> Second Primer

<400> 8
attgatccct ggataatc                                                    18

<210> 9
<211> 18
<212> DNA
<213> Artificial sequence

<220>
<223> Second Primer

<400> 9
attgacccct ggattatg                                                    18

<210> 10
<211> 20
<212> DNA
<213> Artificial sequence

<220>
<223> First Primer

<400> 10
tctgcccgaa ttcgtaaatg                                                  20

<210> 11
<211> 20
<212> DNA
<213> Artificial sequence

<220>
<223> First Primer

<400> 11
cctgcccgaa tttgtaaatg                                                    20


<210> 12
<211> 20
<212> DNA
<213> Artificial sequence

<220>
<223> First Primer

<400> 12
cctgcccgat tatgtaaatg                                                    20


<210> 13
<211> 18
<212> DNA
<213> Artificial sequence

<220>
<223> Second Primer

<400> 13
aattatccag ggatcaat                                                      18


<210> 14
<211> 18
<212> DNA
<213> Artificial sequence

<220>
<223> Second Primer

<400>  14
cattatccag gggtcaat                                                    18

<210>  15
<211>  18
<212>  DNA
<213>  Artificial sequence

<220>
<223>  Second Primer

<400>  15
gattatccag ggatcaat                                                    18

<210>  16
<211>  18
<212>  DNA
<213>  Artificial sequence

<220>
<223>  Second Primer

<400>  16
cataatccag gggtcaat                                                    18

<210>  17
<211>  75
<212>  DNA
<213>  Artificial sequence

<220>
<223>  Cleaving Oligonucleotide

<400> 17
gcaggccatg ttccgctgga tgcgcttcca tgacctcgga ttgtggacag 75
gagatcgcga tcttctgccc gaatt

<210> 18
<211> 75
<212> DNA
<213> Artificial sequence

<220>
<223> Cleaving Oligonucleotide

<400> 18
gcaagccatg ttccgttgga tgcggttcca tgaccttggt ttgtggacag 75
gagatcgcaa tctcctgccc gaatt

<210> 19
<211> 75
<212> DNA
<213> Artificial sequence

<220>
<223> Cleaving Oligonucleotide

<400> 19
gcaggccatg ttccgctgga tgcgcttcca tgatctgagc atgtggacag 75
gggatcgcga tctcctgccc gatta

<210> 20
<211> 75
<212> DNA
<213> Artificial sequence

<220>
<223> Cleaving Oligonucleotide

<400> 20
gcaggctatg ttccgctgga tgcgcttcca tgatctcgga ttgtggacag 75
gagatcgcaa tctcttgccc gaatt

<210> 21
<211> 75
<212> DNA
<213> Artificial sequence

<220>
<223> Cleaving Oligonucleotide

<400> 21
gcaggccatg ttccgctgga tgcgattcca tgatttgagc ttgtggacag 75
gagaccgcga tctcttgccc gatta

<210> 22
<211> 24
<212> DNA
<213> Artificial sequence

<220>
<223> Nucleic Acid Probe Labeled With Intercalating Fluorophore
<400> 22
gtaaatgatg atggcgtcta agga 24

<210> 23
<211> 30
<212> DNA

<213>  Artificial sequence

<220>
<223>  Cleaving Oligonucleotide

<400>  23
aattcgggca gaagatcgcg atctcctgtc                                        30


<210>  24
<211>  30
<212>  DNA
<213>  Artificial sequence

<220>
<223>  Cleaving Oligonucleotide

<400>  24
aattcgggca ggagattgcg atctcctgtc                                        30


<210>  25
<211>  30
<212>  DNA
<213>  Artificial sequence

<220>
<223>  Cleaving Oligonucleotide

<400>  25
taatcgggca ggagatcgcg atcccctgtc                                        30


<210>  26
<211>  30
<212>  DNA

<213>  Artificial sequence

<220>
<223>  Cleaving Oligonucleotide

<400>  26
aattcgggca agagattgcg atctcctgtc                                        30


<210>  27
<211>  18
<212>  DNA
<213>  Artificial sequence

<220>
<223>  Nucleic Acid Probe Labeled With Intercalating Fluorophore

<400>  27
tccttagacg ccatcatc                                                    18


<210>  28
<211>  24
<212>  DNA
<213>  Artificial sequence

<220>
<223>  Cleaving Oligonucleotide

<400>  28
ggcagaagat cgcgatctcc tgtc                                             24


<210>  29
<211>  24
<212>  DNA

<213>  Artificial sequence

<220>
<223>  Cleaving Oligonucleotide

<400>  29
ggcaggagat tgcgatctcc tgtc                                              24


<210>  30
<211>  24
<212>  DNA
<213>  Artificial sequence

<220>
<223>  Cleaving Oligonucleotide

<400>  30
ggcaagagat tgcgatctcc tgtc                                              24


<210>  31
<211>  14
<212>  DNA
<213>  Artificial sequence

<220>
<223>  First Primer

<400>  31
cgaattcgta aatg                                                         14


<210>  32
<211>  14
<212>  DNA

<213> Artificial sequence

<220>
<223> First Primer

<400> 32
cgaatttgta aatg 14

<210> 33
<211> 14
<212> DNA
<213> Artificial sequence

<220>
<223> First Primer

<400> 33
cgattatgta aatg 14

<210> 34
<211> 14
<212> DNA
<213> Artificial sequence

<220>
<223> Second Primer

<400> 34
   atccagggat caat 14

<210> 35
<211> 14
<212> DNA

<213>  Artificial sequence

<220>
<223>  Second Primer

<400>  35
atccaggggt caat                                                          14


<210>  36
<211>  24
<212>  DNA
<213>  Artificial sequence

<220>
<223>  Cleaving Oligonucleotide

<400>  36
aattcgggca gaagatcgcg atct                                               24


<210>  37
<211>  24
<212>  DNA
<213>  Artificial sequence

<220>
<223>  Cleaving Oligonucleotide

<400>  37
taatcgggca ggagatcgcg atcc                                               24


<210>  38
<211>  24
<212>  DNA

<213>  Artificial sequence

<220>
<223>  Cleaving Oligonucleotide

<400>  38
aattcgggca agagattgcg atct                                                24


<210>  39
<211>  23
<212>  DNA
<213>  Artificial sequence

<220>
<223>  First Primer

<400>  39
ttgtggacag gagatcgcta tct                                                 23


<210>  40
<211>  31
<212>  DNA
<213>  Artificial sequence

<220>
<223>  First Primer

<400>  40
ccatgacctc ggattgtgga caggagatcg c                                        31


<210>  41
<211>  31
<212>  DNA

<213> Artificial sequence

<220>
<223> First Primer

<400> 41
ccatgacctt ggtttgtgga caggagatcg c 31

<210> 42
<211> 31
<212> DNA
<213> Artificial sequence

<220>
<223> First Primer

<400> 42
ccatgatttg agcttgtgga caggagaccg c 31

<210> 43
<211> 14
<212> DNA
<213> Artificial sequence

<220>
<223> First Primer

<400> 43
gatgcgcttc catg 14

<210> 44
<211> 14
<212> DNA

<213>  Artificial sequence

<220>
<223>  First Primer

<400>  44
gatgcgattc catg                                                                14

<210>  45
<211>  14
<212>  DNA
<213>  Artificial sequence

<220>
<223>  First Primer

<400>  45
tctgcccgaa ttcg                                                                14

<210>  46
<211>  14
<212>  DNA
<213>  Artificial sequence

<220>
<223>  First Primer

<400>  46
cctgcccgaa tttg                                                                14

<210>  47
<211>  14
<212>  DNA

<213> Artificial sequence

<220>
<223> First Primer

<400> 47
cctgcccgat tatg 14


<210> 48
<211> 18
<212> DNA
<213> Artificial sequence

<220>
<223> Second Primer

<400> 48
aacaatccag ggatcaat 18


<210> 49
<211> 18
<212> DNA
<213> Artificial sequence

<220>
<223> Second Primer

<400> 49
gattatccag ggatcaat 18


<210> 50
<211> 24
<212> DNA

<213> Artificial sequence

<220>
<223> Cleaving Oligonucleotide

<400> 50
ccacaatccg agatcatgga agcg 24

<210> 51
<211> 24
<212> DNA
<213> Artificial sequence

<220>
<223> Cleaving Oligonucleotide

<400> 51
tcatggaagc gcatccagcg gaac 24

<210> 52
<211> 24
<212> DNA
<213> Artificial sequence

<220>
<223> Cleaving Oligonucleotide

<400> 52
tcatggaacc gcatccaacg gaac 24

<210> 53
<211> 24
<212> DNA

<213> Artificial sequence

<220>
<223> Cleaving Oligonucleotide

<400> 53
tcatggaatc gcatccagcg gaac 24

<210> 54
<211> 24
<212> DNA
<213> Artificial sequence

<220>
<223> Cleaving Oligonucleotide

<400> 54
cgcatccagc ggaacatggc ctgc 24

<210> 55
<211> 24
<212> DNA
<213> Artificial sequence

<220>
<223> Cleaving Oligonucleotide

<400> 55
cgcatccaac ggaacatggc ttgc 24

<210> 56
<211> 24
<212> DNA

<213>  Artificial sequence

<220>
<223>  Cleaving Oligonucleotide

<400>  56
cgcatccagc ggaacatagc ctgc                                              24


<210>  57
<211>  18
<212>  DNA
<213>  Artificial sequence

<220>
<223>  Nucleic Acid Probe Labeled With Intercalating Fluorophore

<400>  57
gacgccatca tcatttac                                                     18


<210>  58
<211>  28
<212>  DNA
<213>  Artificial sequence

<220>
<223>  T7 Promoter Sequence

<400>  58
aattctaata cgactcacta tagggaga                                          28